Europäisches Patentamt

European Patent Office

Office européen des brevets

(19)

(11) Veröffentlichungsnummer: **0 190 388**
**B1**

(12) **EUROPÄISCHE PATENTSCHRIFT**

(45) Veröffentlichungstag der Patentschrift:
**20.12.89**

(51) Int. Cl.⁴: **A 61 M 1/00, F 16 L 33/22**

(21) Anmeldenummer: **85106352.9**

(22) Anmeldetag: **23.05.85**

(54) Quetschverbinder.

(30) Priorität: **02.02.85 DE 3503562**
**06.05.85 DE 8513265 U**

(43) Veröffentlichungstag der Anmeldung:
**13.08.86 Patentblatt 86/33**

(45) Bekanntmachung des Hinweises auf die Patenterteilung:
**20.12.89 Patentblatt 89/51**

(84) Benannte Vertragsstaaten:
**AT BE CH DE FR GB IT LI LU NL SE**

(56) Entgegenhaltungen:
**CA-A- 936 438**
**DE-U- 1 619 681**
**FR-A- 2 350 110**
**US-A- 3 241 554**
**US-A- 3 253 594**

(73) Patentinhaber: **Oriplast Gebr. Krayer GmbH, Herstellung und Vertrieb medizinischer und pharmazeutischer Artikel, Hermannstrasse (Industriegebiet), D-6680 Neunkirchen (DE)**

(72) Erfinder: **Krayer, Rolf, Bahnhofstrasse 94, D-6601 Hanweiler (DE)**
Erfinder: **Krayer, Paul, Holbeinstrasse 3, D-6606 Gersweiler (DE)**

(74) Vertreter: **Schönherr, Wolfgang et al, Patentanwälte Wolfgang Schönherr Dipl.-Ing. Karl-Heinz Serwe Hawstrasse 28, D-5500 Trier (DE)**

EP 0 190 388 B1

## Beschreibung

Die Erfindung bezieht sich auf einen Quetschverbinder zum luftdichten Anschluss eines Schlauches, insbesondere eines Drainageschlauches, bestehend aus einem einen hinteren Anschluss für einen Saugschlauch oder dgl. aufweisenden Gehäuse, einer in das vorne offene Gehäuse eingesetzten, etwa zylinderförmigen Hülse aus hochelastischem Werkstoff zur Aufnahme des vorne anzuschliessenden Schlauches und einem mit dem Gehäuse zu verbindenden, die Hülse zusammendrückenden Pressverschluss.

Bekannte derartige Quetschverbinder sind jeweils zum Anschluss eines Drainageschlauches mit einem bestimmten Aussendurchmesser vorgesehen. Soll ein Drainageschlauch mit einem anderen Durchmesser Verwendung finden, so muss ein Quetschverbinder verwendet werden, der auf diesen Durchmesser abgestimmt ist. Dies bedeutet, dass zu jedem Drainageschlauchdurchmesser ein entsprechender Quetschverbinder vorhanden sein muss.

Quetschverbinder zu verwenden, die für Drainageschläuche mit grösserem Durchmesser bestimmt sind, ist nicht möglich, da, wie die Erfahrung gezeigt hat, bei Überschreiten des bestimmungsgemässen Pressbereiches die zylinderförmige Hülse sich unregelmässig verformt, so dass keine dichtende Verbindung hergestellt werden kann.

Die Aufgabe der Erfindung besteht daher darin, einen Quetschverbinder der eingangs genannten Art derart weiterzubilden, dass mit diesem Quetschverbinder Drainageschläuche mit unterschiedlichem Durchmesser luftdicht anschliessbar sind.

Diese Aufgabe wird dadurch gelöst, dass am Gehäuse oder am Pressverschluss sich bei aufgesetztem Pressverschluss in axialer Richtung an der Hülsenaussenseite erstreckende Presstege derart angeordnet sind, dass die Hülse in radialer Richtung zusammengepresst ist.

Vorteilhaft ist die Länge der Presstege etwa gleich der Hülsenlänge in axialer Richtung. Vorzugsweise sind die Presstege zungenartig am Gehäuse oder am Pressverschluss angeordnet. Vorteilhaft sind mindestens drei Presstege am Hülsenumfang angeordnet.

Bei einer vorteilhaften Ausführungsform wirken die Presstege mit einer keilförmig verlaufenden Druckfläche am zugeordneten Gehäuse bzw. am Pressverschluss zusammen. Vorzugsweise sind die zungenförmigen Presstege zu ihrem freien Ende hin keilförmig sich verjüngend ausgebildet.

Vorteilhaft weist die Hülse aus hochelastischem Werkstoff einen sich kegelförmig verjüngenden Abschnitt auf. Vorzugsweise ist der sich verjüngende Abschnitt der Hülse vom Gehäuse zum Pressverschluss hin verjüngend ausgebildet.

Eine andere Ausführungsform eines Quetschverbinders zum luftdichten Anschluss eines Schlauches, insbesondere eines Drainageschlauches, bestehend aus einem einen hinteren Anschluss für einen Saugschlauch od. dgl. aufweisenden Gehäuse, einer in das vorne offene Gehäuse eingesetzten, etwa zylinderförmigen Hülse aus hochelastischem Werkstoff zur Aufnahme des vorne anzuschliessenden Schlauches und einem mit dem Gehäuse zu verbindenden, die Hülse in axialer Richtung zusammendrückenden Pressverschluss ist dadurch gekennzeichnet, dass die Hülse eine in Umfangsrichtung verlaufende nut- oder rillenartige Ausnehmung aufweist.

Vorteilhaft sind mindestens zwei im Abstand zueinander stehende und in Umfangsrichtung verlaufende nut- oder rillenartige Ausnehmungen in der Aussenseite der Hülse vorgesehen.

Vorzugsweise ist der Querschnitt der umlaufenden Ausnehmungen kreisförmig, ellipsenförmig oder keilförmig. Vorzugsweise weisen benachbarte Ausnehmungen unterschiedlichen Querschnitt auf.

Bei einer weiteren vorteilhaften Ausführungsform ist die umlaufende Ausnehmung sich in radialer Richtung der Hülse von ihrem dem Pressverschluss zugeordneten Ende zu ihrem dem Gehäuse zugeordneten Ende hin vertiefend ausgebildet.

Vorteilhaft hat die eine Ausnehmung der Hülse in radialer Richtung der Hülse eine grössere Tiefe als die andere Ausnehmung. Vorzugsweise beträgt die Tiefe der einen Ausnehmung ca. das 1,5fache der Tiefe der anderen Ausnehmung.

Vorteilhaft hat die eine Ausnehmung der Hülse in Längsrichtung der Hülse eine grössere Breite als die andere Ausnehmung. Vorzugsweise beträgt die Breite der einen Ausnehmung der Hülse ca. das 1,5fache der Breite der anderen Ausnehmung.

Vorzugsweise ist die Hülse derart im Gehäuse angeordnet, dass die tiefere und/oder breitere Ausnehmung dem hinteren Anschluss benachbart ist.

Vorzugsweise ist der Pressverschluss mit dem Gehäuse durch eine Schraubverbindung, durch eine mehrere Raststellungen aufweisende Rastverbindung od. dgl. mehr verbindbar.

Die Erfindung ist in den Zeichnungen beispielhaft dargestellt. Es zeigen:

Fig. 1 einen Quetschverbinder mit keilförmigen Presstegen am Gehäuse im Längsschnitt,

Fig. 2 einen Quetschverbinder mit keilförmigen Presstegen am Pressverschluss im Längsschnitt,

Fig. 3 einen Quetschverbinder mit kegelförmig sich verjüngender Hülse im Längsschnitt,

Fig. 4 einen Quetschverbinder mit durch Rastverbindung zu verbindendem Pressverschluss im Längsschnitt.

Fig. 5 den Quetschverbinder nach Fig. 4 im Schnitt,

Fig. 6 einen Quetschverbinder mit einer Hülse mit einer in Umfangsrichtung an der Aussenseite verlaufenden nutartigen Ausnehmung im Längsschnitt,

Fig. 7 eine andere Ausführungsform eines Quetschverbinders mit zwei an der Hülsenaussenseite in Umfangsrichtung im Abstand zueinander verlaufenden nutartigen Ausnehmungen im Längsschnitt,

Fig. 8 einen Quetschverbinder mit einer an der Hülsenaussenseite in Umfangsrichtung verlaufenden nutartigen Ausnehmung mit keilförmigem Querschnitt im Längsschnitt,

Fig. 9 einen Quetschverbinder mit einer in der Innenwandung der Hülse in Umfangsrichtung umlaufend verlaufenden nutartigen Ausnehmung mit keilförmigem Querschnitt im Längsschnitt,

Fig. 10 einen Quetschverbinder mit mehreren, an der Hülseninnenwandung im Abstand zueinander angeordneten, in Umfangsrichtung umlaufenden nutartigen Ausnehmungen im Längsschnitt,

Fig. 11 einen Quetschverbinder im Längsschnitt in auseinandergezogener Darstellung und

Fig. 12 eine andere Ausführungsform eines Quetschverbinders im Längsschnitt in auseinandergezogener Darstellung.

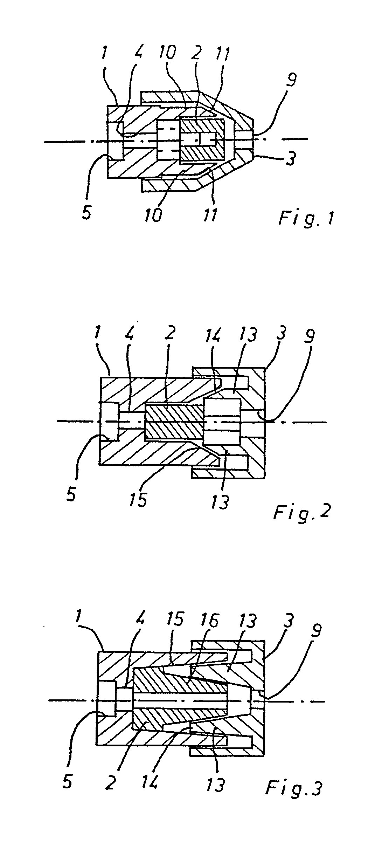

Nach den Fig. 1 bis 12 besteht ein erfindungsgemässer Quetschverbinder aus einem Gehäuse 1, einer in das Gehäuse eingesetzten, etwa zylinderförmigen Hülse 2 aus hochelastischem Werkstoff und einem mit dem Gehäuse 1 zu verbindenden, die Hülse zusammendrückenden Pressverschluss 3.

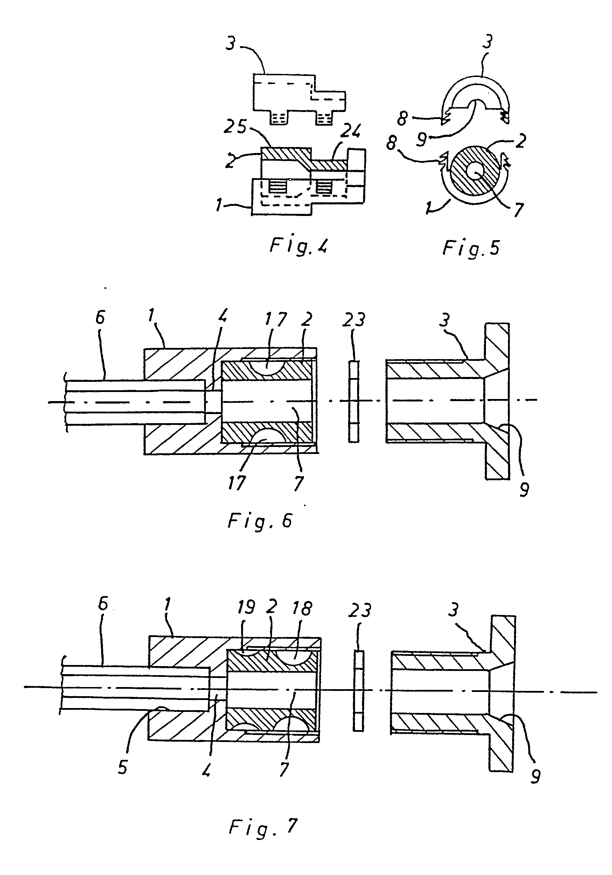

Wie die Fig. 1 bis 12 weiter zeigen, ist das Gehäuse 1 etwa zylinderförmig mit einer inneren, im wesentlichen zylinderischen und in axialer Richtung verlaufenden, durchgehenden Ausnehmung 4 ausgebildet. Am hinteren Ende des Gehäuses 1 ist die Ausnehmung 4 verbreitert als Anschluss 5 für einen Saugschlauch 6 ausgebildet, der in die Ausnehmung 4 eingesetzt ist und mit der Wandung des Gehäuses fest verbunden ist, wie dies in Fig. 6 bis 12 gezeigt ist. An Stelle des Anschlusses 5 kann auch ein anders ausgebildeter Anschluss vorgesehen sein, beispielsweise als Bajonettverschluss od. dgl. mehr.

Die Ausnehmung 4 ist in ihrem vorderen, dem Anschluss 5 abgewandten Abschnitt gleichfalls zylinderförmig verbreitert zur Aufnahme der Hülse 2 aus hochelastischem Werkstoff ausgebildet. Wie die Figuren weiter zeigen, ist die Hülse 2 gleichfalls zylinderförmig, mit einer inneren, in axialer Richtung verlaufenden und durchgehenden Ausnehmung 7 (Fig. 5 bis 12) ausgebildet.

Nach den Fig. 1 bis 3 und 11 ist das Gehäuse 1 mit einem Aussengewinde versehen, auf das der mit einem Innengewinde versehene Pressverschluss 3 aufgeschraubt werden kann. Bei der Auführungsform nach den Fig. 6 bis 10 und 12 ist das Gehäuse 1 im vorderen Bereich der Ausnehmung 4 mit einem Innengewinde versehen, in das der mit einem Aussengewinde versehene Pressverschluss 3 eingeschraubt werden kann. Wie die Fig. 4 und 5 zeigen, kann an Stelle der Schraubgewinde auch ein Rastverschluss 8 vorgesehen sein.

Der Pressverschluss weist nach den Fig. 1 bis 12 eine zentrale Durchtrittsöffnung 9 auf.

Bei Verwendung des erfindungsgemässen Quetschverbinders steht das Gehäuse 1 über den Anschluss 5 und den Saugschlauch 6 mit einer nichtgezeigten Saugvorrichtung in Verbindung. Der zu verbindende Drainageschlauch wird dann durch die Durchtrittsöffnung 9 des Pressverschlusses 3 möglichst weit in die Ausnehmung 7 der Hülse 2 aus hochelastischem Werkstoff eingeschoben und anschliessend der Pressverschluss 3 derart auf das Gehäuse 1 auf- oder eingeschraubt bzw. aufgeschoben, dass die Hülse 2 den eingeschobenen Drainageschlauch dicht umgibt und somit den Drainageschlauch luftdicht mit der nichtgezeigten Saugvorrichtung verbindet.

Nach Fig. 1 weist das Gehäuse 1 in axialer Richtung verlaufende, sich an der Aussenseite der Hülse 2 streckende, zungenartige Pressstege 10 auf, deren Länge etwa gleich der Länge der Hülse 2 ist und die an ihrem vorderen freien Ende 11 sich keilförmig verjüngend ausgebildet sind. Die Pressstege 10, insbesondere ihre keilförmigen Enden 11 wirken mit an dem Pressverschluss 3 angeordneten Druckflächen derart zusammen, dass mit zunehmendem Aufschrauben des Pressverschlusses 3 auf das Gehäuse 1 die Pressstege 10 in radialer Richtung zusammengepresst werden. Dadurch wird auch die Hülse 2 in radialer Richtung zusammengepresst, so dass der in die Ausnehmung 7 der Hülse 2 eingesetzte Drainageschlauch od. dgl. luftdicht abgeschlossen wird.

Bei der Ausführungsform eines Quetschverbinders nach den Fig. 2 und 3 sind die Pressstege 13 mit ihrem keilförmig sich verjüngenden Endabschnitt 14 am Pressverschluss 3 angeordnet, während die zugeordnete Druckfläche 15 am Gehäuse 1 angeordnet ist.

Bei der Ausführungsform eines Quetschverbinders nach Fig. 3 weist die Hülse 2 einen vorderen, sich kegelförmig verjüngenden Abschnitt 16 auf, der mit den keilförmigen Pressstegen 13 des Pressverschlusses 2 zusammenwirkt.

Die Ausnehmung 7 der Hülse 2 kann zur Ausnehmung 4 des Gehäuses 1 hin sich konisch verjüngend ausgebildet sein. Wie die Fig. 4 und 5 zeigen, kann die Hülse 2 auch Abschnitte 24 und 25 mit unterschiedlichem Innen- und Aussendurchmesser aufweisen. Dadurch ist eine besonders gute Anpassung an unterschiedliche Drainageschläuche gegeben.

Bei der Ausführungsform eines Quetschverbinders nach Fig. 6 weist die Hülse 2 an ihrer Aussenseite eine in Umfangsrichtung verlaufende nutartige Ausnehmung 17 mit etwa halbkreisförmigem Querschnitt auf. Bei der Ausführungsform eines Quetschverbinders nach Fig. 7 weist die Hülse an ihrer Aussenseite zwei im Abstand zueinander angeordnete, nutartige Ausnehmungen 18 und 19 mit etwa kreis- bzw. ovalförmigem Querschnitt auf, wobei die dem Anschluss 5 des Gehäuses 1 benachbarte Ausnehmung 19 in radialer Richtung eine geringere Tiefe aufweist, als die nutartige Ausnehmung 18.

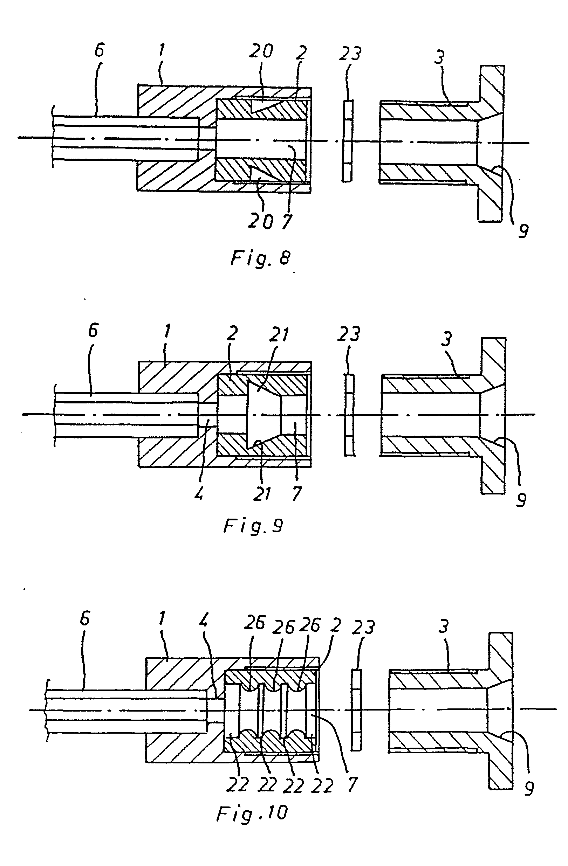

Bei der Ausführungsform eines Quetschverbinders nach Fig. 8 weist die an der Aussenseite der Hülse 2 umlaufend angeordnete nutartige Ausnehmung 20 etwa keilförmigen Querschnitt auf. Die Ausnehmung 20 ist in radialer Richtung der Hülse 2 von ihrem dem Pressverschluss 3 zugeordneten Ende zu ihrem dem Gehäuse 1 zugeordneten Ende hin sich vertiefend ausgebildet.

Bei der Ausführungsform eines Quetschverbinders nach Fig. 9 ist eine keilförmige, in Umfangrichtung umlaufende, nutartige Ausnehmung 21 im Inneren der Hülse 2 angeordnet, wobei der Radius der nutartigen Ausnehmung von dem dem Pressverschluss benachbarten Ende der Hülse zu dem dem Gehäuse benachbarten Ende hin sich vergrössernd ausgebildet ist.

Bei der Ausführungsform eines Quetschverbinders nach Fig. 10 sind im Inneren der Hülse 2 mehrere im Abstand zueinander angeordnete, in Umfangsrichtung umlaufende nutartige Ausnehmungen 22 derart vorgesehen, dass zwischen diesen nutartigen Ausnehmungen Wulste 26 mit etwa halbkreisförmigem Querschnitt bestehen.

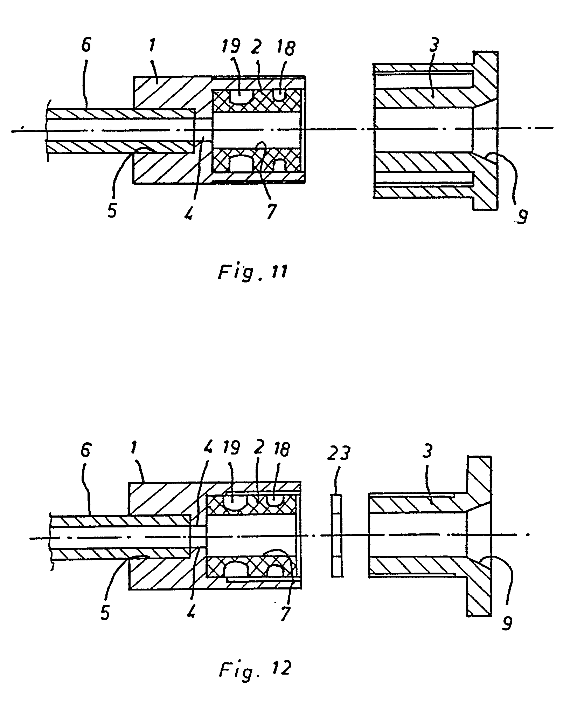

Nach den Fig. 11 und 12 weist die Hülse 2 an ihrer Aussenseite zwei im Abstand zueinander stehende und in Umfangsrichtung der Hülse verlaufende nutartige Ausnehmungen 18 und 19 mit etwa halbkreisförmigem Querschnitt auf. Die dem Anschluss 5 benachbarte Ausnehmung 19 der Hülse 2 weist in radialer Richtung der Hülse ca. die 1,5fache Tiefe und in Längsrichtung der Hülse ca. die 1,5fache Breite der anderen Ausnehmung 18 auf.

Bei Verwendung des erfindungsgemässen Quetschverbinders nach den Fig. 6 bis 12 wird der Pressverschluss 3 ggf. unter Zwischenlage einer Ringscheibe 23 in das Gehäuse 1 eingeschraubt und dadurch die Hülse 2 in axialer Richtung zusammengepresst. Da die Hülse 2 aus hochelastischem Werkstoff sich in radialer Richtung nicht nach aussen ausdehnen kann, wird durch das Zusammenpressen in Folge des Eindrehens des Pressverschlusses 3 der innere Durchmesser der Hülse verringert und so ein luftdichter Anschluss des zu verbindenden Drainageschlauches od. dgl. gewährleistet. Die nutartigen Ausnehmungen 17 bis 22 gewährleisten dabei, dass sich die Hülse derart verformen kann, dass sie sich luftdicht an den zu verbindenden Drainageschlauch anlegt.

Durch den erfindungsgemässen Quetschverbinder ist es möglich, beispielsweise Drainageschläuche von 2 mm (CH. 6) bis 6 mm (CH. 18) luftdicht anzuschliessen.

**Patentansprüche**

1. Quetschverbinder zum luftdichten Anschluss eines Schlauches, insbesondere eines Drainageschlauches, bestehend aus einem einen hinteren Anschluss für einen Saugschlauch (6) od. dgl. aufweisenden Gehäuse (1), einer in das vorne offene Gehäuse eingesetzten, etwa zylinderförmigen Hülse (2) aus hochelastischem Werkstoff zur Aufnahme des vorne anzuschliessenden Schlauches und einem mit dem Gehäuse zu verbindenden, die Hülse zusammendrückenden Pressverschluss (3), dadurch gekennzeichnet, dass am Gehäuse (1) oder am Pressverschluss (3) sich bei aufgesetztem Pressverschluss in axialer Richtung an der Hülsenaussenseite erstreckende Pressstege (10, 13) derart angeordnet sind, dass die Hülse (2) in radialer Richtung zusammengepresst ist.

2. Quetschverbinder nach Anspruch 1, dadurch gekennzeichnet, dass die Länge der Pressstege (10, 13) etwa gleich der Hülsenlänge in axialer Richtung ist, dass die Pressstege (10, 13) zungenartig am Gehäuse (1) oder am Pressverschluss (3) angeordnet sind und dass mindestens drei Pressstege (10, 13) am Hülsenumfang angeordnet sind.

3. Quetschverbinder nach Anspruch 1 oder 2, dadurch gekennzeichnet, dass die Pressstege (10, 13) mit einer keilförmig verlaufenden Druckfläche (15) am zugeordneten Gehäuse (1) bzw. am Pressverschluss (3) zusammenwirken und dass die zungenförmigen Pressstege (10, 13) zu ihrem freien Ende (11, 14) hin keilförmig sich verjüngend ausgebildet sind.

4. Quetschverbinder nach einem der Ansprüche 1 bis 3, dadurch gekennzeichnet, dass die Hülse (2) aus hochelastischem Werkstoff einen sich kegelförmig verjüngenden Abschnitt (16) aufweist und dass der sich verjüngende Abschnitt (16) der Hülse (2) vom Gehäuse (1) zum Pressverschluss (3) hin sich verjüngend ausgebildet ist.

5. Quetschverbinder zum luftdichten Anschluss eines Schlauches, insbesondere eines Drainageschlauches, bestehend aus einem einen hinteren Anschluss für einen Saugschlauch (6) od. dgl. aufweisenden Gehäuse (1), einer in das vorne offene Gehäuse eingesetzten, etwa zylinderförmigen Hülse (2) aus hochelastischem Werkstoff zur Aufnahme des vorne anzuschliessenden Schlauches und einem mit dem Gehäuse zu verbindenden, die Hülse in axialer Richtung zusammendrückenden Pressverschluss (3), dadurch gekennzeichnet, dass die Hülse (2) eine in Umfangsrichtung verlaufende nut- oder rillenartige Ausnehmung (17 bis 22) aufweist.

6. Quetschverbinder nach Anspruch 5, dadurch gekennzeichnet, dass mindestens zwei im Abstand zueinander stehende und in Umfangsrichtung verlaufende nut- oder rillenartige Ausnehmungen (18, 19 und 22) in der Aussenseite der Hülse (2) vorgesehen sind.

7. Quetschverbinder nach Anspruch 5 oder 6, dadurch gekennzeichnet, dass der Querschnitt der umlaufenden Ausnehmung (17 bis 22) halbkreisförmig, ellipsenförmig oder keilförmig ist und dass benachbarte Ausnehmungen (18, 19) unterschiedlichen Querschnitt aufweisen.

8. Quetschverbinder nach Anspruch 7, dadurch gekennzeichnet, dass die umlaufende Ausnehmung (20, 21) sich in radialer Richtung der Hülse (2) von ihrem dem Pressverschluss (3) zugeordneten Ende zu ihrem dem Gehäuse (1) zugeordneten Ende hin vertiefend ausgebildet ist.

9. Quetschverbinder nach einem der Ansprüche 6 bis 8, dadurch gekennzeichnet, dass die eine Ausnehmung (19) der Hülse (2) in radialer Richtung der Hülse eine grössere Tiefe als die andere Ausnehmung (18) hat.

10. Quetschverbinder nach einem der Ansprüche 1 bis 9, dadurch gekennzeichnet, dass die Hülse (2) im Innern konisch sich verjüngend ausgebildet ist.

11. Quetschverbinder nach einem der Ansprüche 1 bis 10, dadurch gekennzeichnet, dass die Hülse (2) Abschnitte (24, 25) unterschiedlichen Innendurchmessers und/oder Aussendurchmessers aufweist.

12. Quetschverbinder nach einem der Ansprüche 1 bis 11, dadurch gekennzeichnet, dass der Pressverschluss (3) mit dem Gehäuse (1) durch eine Schraubverbindung, durch eine mehrere Raststellungen aufweisende Rastverbindung (8) od. dgl. verbindbar ist.

## Claims

1. Squeeze connector for airtight connection of a hose, in particular a drainage hose, comprising a housing (1) including a rear connection for a suction hose (6) or similar, an approximately cylindrical sleeve (2) of resilient material for accommodating the hose to be connected at the front inserted into the housing which is open at the front, and a squeeze seal which is connected with the housing and squeezes the sleeve together, characterised in that at the housing (1) or at the pressure seal (3) with pushed-on pressure seal the pressure tongues (10, 13) extending axially along the sleeve's outer circumference are disposed in such a way that the sleeve (2) is squeezed together in radial direction.

2. Squeeze connector according to claim 1, characterised in that the length of the pressure tongues (10, 13) is approximately equal to the length of the sleeve in axial direction; that the pressure tongues (10, 13) are disposed tongue-like at the housing (1) or at the pressure seal (3); and that at least three pressure tongues (10, 13) are disposed at the sleeve circumference.

3. Squeeze connector according to claim 1 or 2, characterised in that the pressue tongues (10, 13) cooperate with a wedge-shaped pressure surface (15) at the respective housing (1) or at the pressure seal (3) respectively; and that the pressure tongues (10, 13) are wedge-shaped and tapered in the direction of their free ends (11, 14).

4. Squeeze connector according to one of the claims 1 to 3, characterised in that the sleeve (2) of resilient material has a wedge-shaped tapered section (16); and that the tapered section (16) of the sleeve (2) is tapered from the housing (1) to the pressure seal (3).

5. Squeeze connector for airtight connection of a hose, in particular a drainage hose, comprising a housing (1) including a rear connection for a suction hose (6) or similar, an approximately cylindrical sleeve (2) of resilient material for accommodating the hose to be connected at the front inserted into the housing which is open at the front, and a squeeze seal (3) which is connected with the housing and squeezes the sleeve together, characterised in that the sleeve (2) has a notch- or groove-like indentation (17 to 22) in the circumferencial direction.

6. Squeeze connector according to claim 5, characterised by the provision of at least two notch- or groove-like indentations (18, 19 and 22) disposed at a distance from each other and in the circumferential direction at the outside of the sleeve (2).

7. Squeeze connector according to claim 5 or 6, characterised by the cross-section of the circumferential indentation (17 to 22) being semicircular, elliptical or wedge-shaped; and by adjacent indentations (18, 19) having different cross-sections.

8. Squeeze connector according to claim 7, characterised by the circumferential indentation (20, 23) being hollowed out in the radial direction of the sleeve (2) from its end nearest the squeeze seal (3) to the end nearest the housing (1).

9. Squeeze connector according to one of the claims 6 to 8, characterised by the indentation (19) of the sleeve (2) having a depth in the radial direction of the sleeve which is deeper than the other indentation (18).

10. Squeeze connector acording to one of the claims 1 to 9, characterised by the sleeve (2) being constructed to be internally conically tapering.

11. Squeeze connector according to one of the claims 1 to 10, characterised by the sleeve (2) having sections (24, 25) of different inside diameters and/or outside diameters.

12. Squeeze connector according to one of the claims 1 to 11, characterised by the pressure seal (3) being connectable with the housing (1) by way of a screw connection, by a ratchet connection comprising several ratchet positions, or similar means.

## Revendications

1. Raccord compressible pour le raccordement étanche à l'air d'un tuyau, en particulier d'un tuyau de drainage, constitué d'un boîtier (1) présentant un raccord arrière pour un tuyau d'aspiration (6) ou analogue, une douille (2) de forme sensiblement cylindrique, insérée dans le boîtier ouvert à l'avant et réalisée en une matière hautement élastique pour la réception du tuyau à raccorder à l'avant, et un obturateur (3) de pression comprimant la douille et devant être raccordé au boîtier, caractérisé par le fait que sur le boîtier (1) ou sur l'obturateur (3), lorsque ledit obturateur est monté, des nervures de compression (10, 13) s'étendant en direction axiale sur le côté extérieur de la douille sont disposées de telle sorte que ladite douille (2) est comprimée dans le sens radial.

2. Raccord compressible selon la revendication 1, caractérisé par le fait que la longueur des nervures de compression (10, 13) est sensiblement égale à la longueur de la douille dans le sens axial, que les nervures (10, 13) sont disposées à la manière de languettes sur le boîtier (1) ou sur l'obturateur (3) et qu'au moins trois nervures (10, 13) sont disposées sur le pourtour de la douille.

3. Raccord compressible selon la revendication 1 ou 2, caractérisé par le fait que les nervures de compression (10, 13) coopèrent avec une surface de compression angulaire (15) sur le boîtier (1) ou l'obturateur (3) qui leur est associé et que lesdites nervures (10, 13) en forme de languettes sont réalisées sous forme de cônes dont la pointe est orientée vers leur extrémité libre (11, 14).

4. Raccord compressible selon l'une des revendications 1 à 3, caractérisé par le fait que la douille (2) en matière hautement élastique présente une section (16) angulaire et que ladite section (16) de la douille (2) s'imincit en allant du boîtier (1) vers l'obturateur (3).

5. Raccord compressible pour le raccordement étanche à l'air d'un tuyau en particulier d'un tuyau de drainage constitué d'un boîtier (1) présentant un raccord arrière pour un tuyau d'aspiration (6) ou analogue, une douille (2) de forme sensiblement cylindrique, insérée dans le boîtier ouvert à l'avant et réalisée en une matière hautement élastique pour la réception du tuyau à raccorder à l'avant, et un obturateur (3) de pression comprimant la douille et devant être raccordé au boîtier, caractérisé par le fait que la douille (2) présente un évidement (17 à 22) s'étendant dans le sens périphérique et ayant la forme d'une rainure ou d'une gorge.

6. Raccord compressible selon la revendication 5, caractérisé par le fait qu'au moins deux évidements (18, 19 et 22) en forme de rainure ou de gorge, placés à une certaine distance mutuelle et s'étendant dans le sens périphérique, sont prévus dans le côté extérieur de la douille (2).

7. Raccord compressible selon la revendication 5 ou 6, caractérisé par le fait que la section de l'évidement périphérique (17 à 22) est de forme semi-circulaire, elliptique ou angulaire et que des évidement adjacents (18, 19) présentent des sections différentes.

8. Raccord compressible selon la revendication 7, caractérisé par le fait que l'évidement périphérique (20, 21) est réalisé de manière à s'approfondir dans le sens radial de la douille (2) ente son extrémité associée à l'obturateur (3) et son extrémité associée au boîtier (1).

9. Raccord compressible selon l'une quelconque des revendications 6 à 8, caractérisé par le fait que, vue en direction radiale de la douille, la profondeur de l'un des évidements (19) de ladite douille (2) est supérieure à celle de l'autre évidement (18).

10. Raccord compressible selon l'une quelconque des revendications 1 à 9, caractérisé par le fait que l'intérieur de la douille (2) est de forme conique.

11. Raccord compressible selon l'une quelconque des revendications 1 à 10, caractérisé par le fait que la douille (2) présente des sections (24, 25) ayant des diamètres intérieur et/ou extérieur différents.

12. Raccord compressible selon l'une quelconque des revendications 1 à 11, caractérisé par le fait que l'obturateur de compressoin (3) peut être raccordé au boîtier (1) par un boulonnage, par un raccord à baïonnette (8) présentant plusieurs encoches ou par un raccord analogue.

Fig. 1

Fig. 2

Fig. 3

Fig. 4

Fig. 5

Fig. 6

Fig. 7

Fig. 8

Fig. 9

Fig .10

Fig. 11

Fig. 12